# EUROPEAN PATENT APPLICATION

(11) **EP 4 464 362 A1**
(43) Date of publication of application: **20.11.2024**
(21) Application number: 23740276.3
(22) Date of filing: 12.01.2023
(51) Int. Cl.: A61M 25/10, A61B 18/04

(54) **LIQUID CONTROL SYSTEM, BALLOON CATHETER ASSEMBLY, BALLOON CATHETER SYSTEM, AND METHOD FOR USING BALLOON CATHETER SYSTEM**

(30) Priority: 12.01.2022 JP 2022003076
(71) Applicant: Toray Industries, Inc., Tokyo 103-8666 (JP)
(72) Inventor: OKA Yumi, Otsu-shi, Shiga 520-2141 (JP); TERASAKA Hiroyuki, Otsu-shi, Shiga 520-2141 (JP)
(74) Representative: Kador & Partner Part mbB
(86) International application number: PCT/JP2023/000513
(87) International publication number: WO 2023/136268

(57) **Abstract**

A liquid control system supplies and discharges a liquid to and from a balloon. The liquid control system includes a syringe pump including a syringe, a liquid flow member coupling the syringe and the balloon catheter, a reservoir coupled to the syringe by the liquid flow member, and a liquid flow controller that controls flow of the liquid in the liquid flow member. The liquid flow member is connected to the syringe from above, and is connected to the reservoir from below. The liquid flow controller switches a first state in which communication between the syringe and the reservoir is blocked and communication between the syringe and the balloon catheter is allowed, and a second state in which the communication between the syringe and the balloon catheter is blocked and the communication between the syringe and the reservoir is allowed.

## Description

### Technical Field

The present invention relates to a liquid control system, a balloon catheter assembly, a balloon catheter system, and a using method of a balloon catheter system.

### Background Art

As disclosed in Patent Documents 1 to 4, treatment using a balloon catheter is known. The balloon catheter includes a shaft having a lumen, and a balloon fixed on a distal end part of the shaft. The lumen is used as a liquid flow path. The balloon is inflated by supplying thereto a liquid through the lumen. The balloon catheter is used to treat arrhythmias, for example.

In the balloon catheter disclosed in Patent Document 1, the shaft includes two lumens. One lumen functions as a liquid flow path through which a liquid is supplied to the balloon. The other lumen functions as a liquid flow path through which the liquid is discharged from the balloon. Circulation of the liquid through the two lumens can avoid the liquid in the balloon becoming excessively hot, and can inhibit a temperature of the liquid in the balloon from varying. However, the shaft including the two lumens is thick. The thick balloon catheter is a burden on a patient when it is inserted into his/her body.

In the balloon catheters disclosed in Patent Documents 2 to 4, the shaft includes one lumen as a liquid flow path. The balloon catheter is connected to an agitator serving as a vibrator. The agitator repeatedly supplies a liquid to the lumen of the shaft and discharges the liquid therefrom. This vibrates the liquid in the balloon to agitate the liquid in the balloon. This can avoid the liquid in the balloon becoming excessively hot, and can inhibit a temperature of the liquid in the balloon from varying. In this balloon catheter, it is necessary to discharge gas from the lumen serving as a liquid flow path before treatment. If gas remains in the liquid flow path, expansion and contraction of the gas prevent the liquid in the balloon from being sufficiently agitated. In addition, bubbles remaining in the balloon may inhibit temperature transmission to a tissue.

As described in Patent Document 2, paragraph 0029 and Fig. 1, and Patent Document 3, paragraph 0033 and Fig. 1, the conventional balloon catheter system uses a syringe for discharging gas from the liquid flow path. Patent Document 4 describes a gas discharge method using a syringe. Specifically, a liquid is supplied from the syringe to the balloon catheter, and then the liquid is collected together with gas from the balloon catheter to the syringe. The gas discharged from the balloon catheter is collected in the syringe.

Patent document 1: JP 2020-513912 A
Patent document 2: JP 2010-233810 A
Patent document 3: JP 2010-240004 A
Patent document 4: WO 2014/122759 A

In order to completely discharge the gas from the syringe, the supply of the liquid to the balloon catheter and the discharge of the liquid from the balloon catheter may be repeated multiple times. In this example, the gas collected in the syringe is discharged each time outside a system composed of the syringe, the liquid flow path, and the balloon catheter.

The balloon is inflated to an appropriate size in accordance with a body of a patient upon treatment. As shown in Patent Document 2, paragraph 0029, the syringe used for discharging gas is used to supply a liquid to the balloon catheter for inflating the balloon to a desired size. It is necessary to discharge gas from the syringe to the outside of the system, before a predetermined amount of a liquid is sent from the syringe toward the balloon catheter.

However, the process of discharging the gas in the syringe out of the system is complicated for a balloon catheter user because it involves switching of a valve and operation of the syringe.

In addition, in Patent Documents 2 to 4, the syringe used for discharging gas is communicated with the liquid flow path of the catheter via a three-way stopper cock or the like, separately from the agitator. Thus, gas in the liquid flow path from the agitator up to the balloon catheter must be discharged separately. Thus, the preparation before use of the balloon catheter system including the balloon catheter is further complicated.

### Disclosure of The Invention

The present invention has been made in view of the above circumstances. The object of the present invention is to simplify the preparation before use of the balloon catheter system.

A liquid control system of the present disclosure supplies a liquid to a balloon catheter and discharges the liquid from the balloon catheter, the liquid control system comprising: a syringe pump including a syringe including a cylinder and a piston that is relatively movable with respect to the cylinder, a holding unit holding the syringe, and a drive that relatively moves the piston with respect to the cylinder; a liquid flow member coupling the syringe and the balloon catheter; a reservoir coupled to the syringe by the liquid flow member; and a liquid flow controller that controls flow of the liquid in the liquid flow member; wherein: the liquid flow member is connected to the syringe from above, and is connected to the reservoir from below; and the liquid flow controller switches a first state in which communication between the syringe and the reservoir is blocked and communication between the syringe and the balloon catheter is allowed (achieved), and a second state in which the communication between the syringe and the balloon catheter is blocked and the communication between the syringe and the reservoir is allowed (achieved).

In the liquid control system of the present disclosure, the liquid flow member may include a first line part connected to the balloon catheter, and a second line part connected to the reservoir; the liquid flow member including the second line part may be connected to the cylinder from above; and the second line part may be connected to the reservoir from below.

In the liquid control system of the present disclosure, the liquid flow controller may include a first pinch valve that opens and closes the first line part, and a second pinch valve that opens and closes the second line part.

In the liquid control system of the present disclosure, the liquid flow controller may further include a three-way valve connected to both the first line part and the second line part.

In the liquid control system of the present disclosure, the liquid flow member may further include a joint line part coupling the three-way valve and the cylinder.

In the liquid control system of the present disclosure, the reservoir may include a reservoir syringe including a cylinder connected to the second line part, and a piston that is relatively movable with respect to the cylinder; and the cylinder of the reservoir syringe may be positioned below the piston of the reservoir syringe, and the piston of the reservoir syringe may be relatively moved upward and downward with respect to the cylinder of the reservoir syringe.

In the liquid control system of the present disclosure, the holding unit may hold the syringe such that the cylinder is positioned above the piston; and the drive may relatively move the piston upward and downward with respect to the cylinder.

In the liquid control system of the present disclosure, a connection position between the liquid flow member and the syringe pump may be positioned above a connection position between the liquid flow member and the balloon catheter.

In the liquid control system of the present disclosure, a connection position between the liquid flow member and the reservoir may be positioned above a connection position between the liquid flow member and the syringe pump.

In the liquid control system of the present disclosure, the liquid flow member may include a first line part connected to the balloon catheter, a second line part connected to the reservoir, and a joint line part connected to the syringe; the first line part and the second line part may branch off from the joint line part; and a lowermost portion of the second line part may be located at the same vertical position as an uppermost portion of the first line part, or at a position vertically above the uppermost portion of the first line part.

In the liquid control system of the present disclosure, the liquid flow member may include a first line part connected to the balloon catheter, a second line part connected to the reservoir, and a joint line part connected to the syringe; the first line part and the second line part may branch off from the joint line part; and a lowermost portion of the second line part may be located at the same vertical position as an uppermost portion of the joint line part, or at a position vertically above the uppermost portion of the joint line part.

In the liquid control system of the present disclosure, the drive may be capable of adjusting a relative movement amount of the piston and the cylinder.

In the liquid control system of the present disclosure, the drive may drive the piston such that a movement speed at which the piston is pushed into the cylinder and a movement speed at which the piston is drawn out from the cylinder differ from each other.

A balloon catheter system according to the present disclosure comprises any one of the aforementioned liquid control system according to the present disclosure, and the balloon catheter.

A using method according to the present disclosure is a using method of the aforementioned balloon catheter system according to the present disclosure, comprising: a supply step in which the liquid flow member is placed in the first state, and the liquid in the syringe is supplied to the balloon catheter by pushing the piston into the cylinder; a discharge step in which the liquid flow member is placed in the first state, and the liquid and gas in the balloon catheter are discharged from the balloon catheter by drawing out the piston from the cylinder; and a transport step in which the liquid flow member is placed in the second state, and the gas is sent to the reservoir by pushing the piston into the cylinder.

In the using method according to the present disclosure, the supply step, the discharge step, and the transport step may be repeated in this order.

The using method according to the present disclosure may comprise a second discharge step following the transport step, in which the liquid flow member is placed in the second state, and the liquid is discharged from the reservoir by drawing out the piston from the cylinder, with the gas remaining in the reservoir.

In the using method according to the present disclosure, the supply step, the discharge step, the transport step, and the second discharge step may be repeated in this order.

The using method according to the present disclosure may comprise an inflation step following the transport step, in which the liquid flow member is placed in the first state, and a preset amount of the liquid is supplied to the balloon catheter to inflate the balloon by pushing the piston into the cylinder by means of the drive.

The using method according to the present disclosure may comprise a heating step following the inflation step, in which the liquid flow member is placed in the first state, and the liquid in the balloon is heated while supply of the liquid to the balloon catheter and discharge of the liquid therefrom are repeated by moving the piston to and from the cylinder by means of the drive.

In the using method according to the present disclosure, the drive may adjust a relative movement amount of the piston and the cylinder.

In the using method according to the present disclosure, a relative movement speed of the piston with respect to the cylinder in the supply step may be slower than a relative movement speed of the piston with respect to the cylinder in the discharge step.

A balloon catheter assembly according to the present disclosure comprises: a balloon catheter; a liquid flow member connected to the balloon catheter; a syringe including a cylinder coupled to the balloon catheter by the liquid flow member, and a piston that is relatively movable with respect to the cylinder; and a reservoir coupled to the syringe by the liquid flow member; wherein: the liquid flow member enables communication between the syringe and the balloon catheter, and enables communication between the syringe and the reservoir; and the liquid flow member is capable of being connected to the syringe from above, and is capable of being connected to the reservoir from below.

The present invention can simplify the preparation before use of the balloon catheter system.

### Brief Description of The Drawings

Fig. 1 is a view for describing an embodiment, showing a balloon catheter system, a balloon catheter assembly, a balloon catheter, and a liquid control system.
Fig. 2 is a view showing a distal end portion of the balloon catheter of Fig. 1, with the balloon being inflated.
Fig. 3 is a view showing the distal end portion of the balloon catheter of Fig. 1, with the balloon being deflated and stretched.
Fig. 4 is a view for describing an operation of the liquid control system and a using method of the balloon catheter system.
Fig. 5 is a view for describing the operation of the liquid control system and the using method of the balloon catheter system.
Fig. 6 is a view for describing the operation of the liquid control system and the using method of the balloon catheter system.
Fig. 7 is a view for describing the operation of the liquid control system and the using method of the balloon catheter system.
Fig. 8 is a view for describing the operation of the liquid control system and the using method of the balloon catheter system.
Fig. 9 is a view for describing the operation of the liquid control system and the using method of the balloon catheter system.
Fig. 10 is a view for describing the operation of the liquid control system and the using method of the balloon catheter system.
Fig. 11A is a view showing a modification example of a reservoir included in the liquid control system.
Fig. 11B is a view showing another modification example of the reservoir.
Fig. 11C is a view showing yet another modification example of the reservoir.
Fig. 12 is a view showing a modification example of a liquid flow member included in the liquid control system.
Fig. 13 is a view showing another modification example of the liquid flow member included in the liquid control system.
Fig. 14A is a view showing an example of a cylinder of a syringe that may be included in a syringe pump.
Fig. 14B is a view showing a placement example of the cylinder shown in Fig. 14A.
Fig. 15A is a view showing another example of the cylinder of the syringe that may be included in the syringe pump.
Fig. 15B is a view showing a placement example of the cylinder shown in Fig. 15A.

### Mode for Carrying out the Invention

One embodiment of the present invention is described below with reference to the drawings. In the drawings attached to the specification, a scale dimension, an aspect ratio and so on are changed and exaggerated from the actual ones, for the convenience of easiness in illustration and understanding. A configuration, etc. shown in some drawings may be omitted in other drawings.

In the specification, terms specifying shapes, geometric conditions and their degrees, and specific values are not limited to their strict definitions, but construed to include a range capable of exerting a similar function.

A balloon catheter system 10 is used for catheter ablation therapy. The catheter ablation therapy is a treatment method to ablate a target site in a body. The catheter ablation therapy may be used to treat arrhythmia due to atrial fibrillation, endometriosis, cancer, etc.

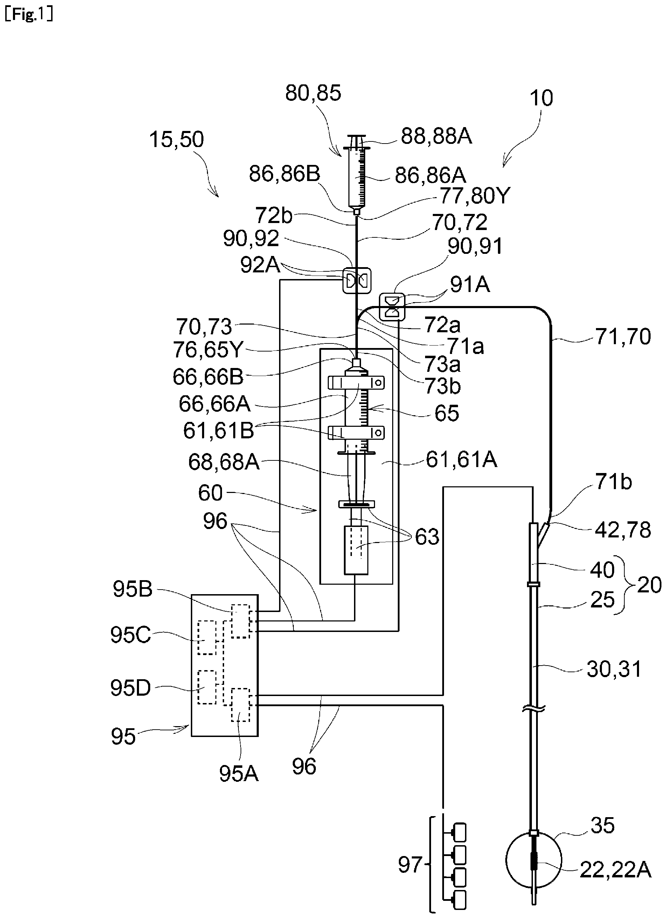

As shown in Fig. 1, the balloon catheter system 10 includes a balloon catheter 20 and a liquid control system 50. The balloon catheter 20 includes a shaft member 30 to be inserted into a body, and a balloon 35 connected to the shaft member 30. The balloon catheter 20 includes a liquid flow path leading to an inside of the balloon 35. The liquid control system 50 is connected to the balloon catheter 20. The liquid control system 50 supplies a liquid to the liquid flow path 28 of the balloon catheter 20, and discharges the liquid from the liquid flow path 28. The liquid includes not only a liquid in the strict sense of the word but also a suspension liquid. An example of the liquid is a dilute contrast agent. The dilute contrast agent may include a contrast agent and saline.

When the balloon catheter 20 is inserted into a body, the balloon is deflated and stretched in a longitudinal direction of the balloon catheter. The balloon catheter 20 inserted into the body is supplied with a liquid through the liquid flow path 28, so that the balloon is inflated. During catheter ablation therapy, the inflated balloon 35 is brought into contact with a target site in the body. Since the liquid in the balloon 35 is heated, the target site can be ablated. The balloon catheter 20 can circumferentially bring the balloon 35 into contact with a circumferential target site, for example, a connection point of a vein to the atrium. Thus, the circumferential target site can be ablated at once.

In this embodiment, the balloon catheter system 10 including the balloon catheter 20 is designed such that its preparation before use is simplified. The balloon catheter system 10, the balloon catheter assembly 15, and the liquid control system 50 according to one embodiment are described herebelow, with reference to a specific example shown in the drawings.

The balloon catheter 20 included in the balloon catheter system 10 is described first. A conventional balloon catheter can also be applied to this embodiment. The balloon catheter 20 is described with reference to a specific example shown in Figs. 1 to 3.

The balloon catheter 20 shown in Figs. 1 to 3 includes a catheter body 25 and a handle 40. The handle 40 is attached to a proximal end of the catheter body 25. The catheter body 25 includes a heating member 22, in addition to the aforementioned shaft member 30 and the balloon 35. The heating member 22 is used for heating a liquid in the balloon 35. The shaft member 30 includes an outer shaft (shaft, first shaft) 31 and an inner shaft (second shaft) 32.

The outer shaft 31 has a lumen 31L defining the liquid flow path 28. The outer shaft 31 is provided with the single lumen 31L. The lumen 31L may extend from a distal end 31a of the outer shaft 31 to a proximal end thereof. The inner shaft 32 is located in the lumen 31L of the outer shaft 31. Namely, the shaft member 30 has a double cylinder structure.

The "distal" side used for respective structures of the balloon catheter 20 means a side away from the handle 40 and an operator (surgeon) of the handle 40 along a longitudinal direction LD of the catheter body 25, in other words, a distant end side. The "proximal" side used for respective structures of the balloon catheter 20 means a side close to the handle 40 and an operator (surgeon) of the handle 40 along the longitudinal direction LD of the catheter body 25, in other words, a near end side. The longitudinal direction LD of the catheter body 25 is specified as a direction along which a center axis of the shaft member 30 extends.

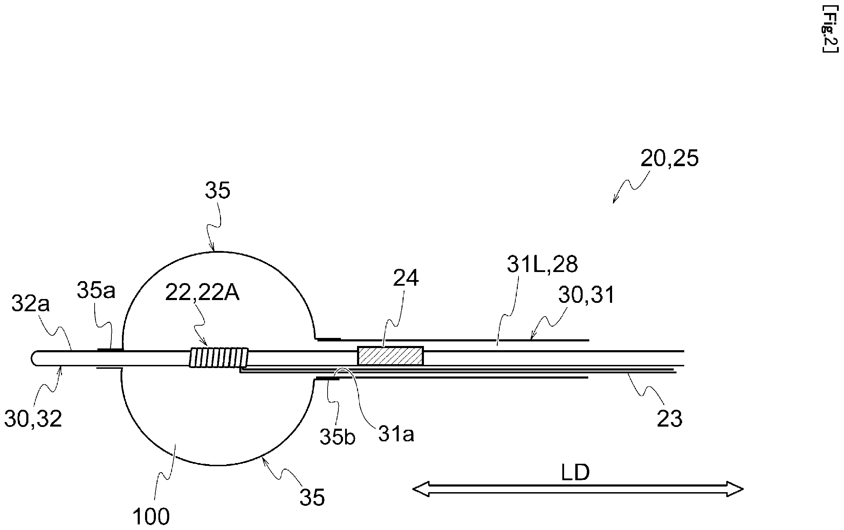

The outer shaft 31 and the inner shaft 32 both have a cylindrical shape, typically, a circular cylindrical shape. Thus, the inner shaft 32 may also have a lumen. The lumen formed by the inner shaft 32 may be used for insertion of a guidewire, not shown. An inner dimension (inner width, internal diameter) of the lumen 31L of the outer shaft 31 is larger than an outer dimension (outer width, external diameter) of the inner shaft 32. This dimension difference forms the liquid flow path 28. As shown in Fig. 2, the liquid flow path 28 leads into the balloon 35. The liquid flow path 28 extends into the handle 40.

Length of the outer shaft 31 and the inner shaft 32 may be respectively 500 mm or more and 1700 mm or less, or may be 600 mm or more and 1200 mm or less. The outer shaft 31 and the inner shaft 32 are preferably made by using a flexible material with excellent anti-thrombogenic properties. Examples of the flexible material with excellent anti-thrombogenic properties include fluoropolymers, polyamides, polyurethane-based polymers, polyimides or the like. The material forming the outer shaft 31 may be a layered body of different flexible materials in order to achieve both sliding ability with the inner shaft 32 and attachability or heat weldability with the balloon 35.

An external diameter of the outer shaft 31 may be 2.5 mm or more and 4.0 mm or less. An internal diameter of the outer shaft 31 may be 2.5 mm or more and 3.5 mm or less. An external diameter of the inner shaft 32 may be 1.2 mm or more and 1.8 mm or less. An internal diameter of the inner shaft 32 may be 0.9 mm or more and 1.5 mm or less.

The balloon 35 is connected to the outer shaft 31 and the inner shaft 32. The balloon 35 is inflatable when it is filled with a liquid, and is deflatable when the liquid is discharged therefrom. The balloon 35 may have a shape to fit a target site to be treated (e.g., blood vessel). For example, the balloon 35 adapted to be a pulmonary venous junction of a left atrium has a spherical shape having a diameter of 15 mm or more and 40 mm or less.

A film thickness of the balloon 35 may be 10 µm or more and 200 µm or less A material of the balloon 35 may be an elastic material with excellent anti-thrombogenic properties. Specifically, the material of the balloon 35 may be a polyurethane-based polymeric material or the like. Examples of the polyurethane-based polymeric material applicable to the balloon 35 include thermoplastic polyether urethane, polyether polyurethane urea, fluorinated polyether urethane urea, polyether polyurethane urea resin, or polyether polyurethane urea amide.

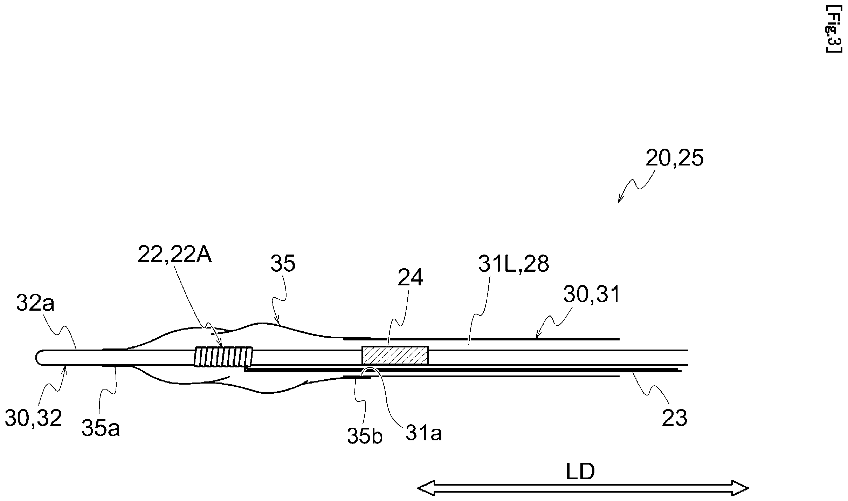

As shown in Fig. 2 and 3, a distal end (distant end) 35a of the balloon 35 may be fixed on a distal end (distant end) 32a of the inner shaft 32. A proximal end (near end) 35b of the balloon 35 may be fixed on a distal end (distant end) 31a of the outer shaft 31. The balloon 35 and the outer shaft 31 or the inner shaft 32 may be connected by adhesion or heat welding.

When the outer shaft 31 and the inner shaft 32 are relatively moved to each other in the longitudinal direction LD, the balloon 35 connected to the outer shaft 31 and the inner shaft 32 is deformed. In the illustrated example, dimensions of the balloon 35 in the longitudinal direction LD can be adjusted by the relative movement of the outer shaft 31 and the inner shaft 32. As shown in Fig. 3, when the inner shaft 32 is relatively moved with respect to the outer shaft 31 distally in the longitudinal direction LD, the balloon 35 is stretched in the longitudinal direction LD to become taut. In the illustrated example, the distal movement range of the inner shaft 32 with respect to the outer shaft 31 in the longitudinal direction LD is restricted by the balloon 35. When the inner shaft 32 is relatively moved with respect to the outer shaft 31 from the position shown in Fig. 3 proximally in the longitudinal direction LD, the balloon 24 becomes loosened. By introducing a liquid into the loosened balloon 35, the balloon 35 can be inflated as shown in Fig. 2. Namely, the dimensions of the balloon 35 in the longitudinal direction LD can be adjusted by the relative movement of the outer shaft 31 and the inner shaft 32.

Next, the heating member 22 is described. The heating member 22 is located in the balloon 35. The heating member 22 is a member for heating a liquid filled in the balloon 35. The heating member 22 shown in Figs. 2 and 3 is a coil electrode 22A. By performing high-frequency current conduction (high-frequency energization) to the coil electrode 22A, a high-frequency current flows between it and a counter electrode (return pad) 97 (see Fig. 1) located outside the body. At this time, the liquid generates Joule heat between the coil electrode 22A and the counter electrode 97. The counter electrode 97 is located on a back of a patient, for example.

As shown in Figs. 2 and 3, the coil electrode 22A may be provided on the inner shaft 32 extending in the balloon 35. The coil electrode 22A may be formed by a conducting wire wound around the inner shaft 32. In the illustrated example, the coil electrode 22A is electrically connected to a lead wire 23 for high-frequency current conduction. The lead wire 23 extends in the liquid flow path 28, which is the lumen between the outer shaft 31 and the inner shaft 32, up to the handle 40. A specific example of the coil electrode 22A forming the heating member 22 may be a coil electrode that is made as follows. An insulation-coated lead wire used as the lead wire 23 is stripped of its coat. Then, the lead wire is wound around the inner shaft 32 to provide a coil electrode. Since such a coil electrode 22A is integrally formed with the lead wire 23, trouble such as disconnection can be avoided.

Diameters of the coil electrode 22A and the lead wire 23 may be 0.1 mm or more and 1 mm or less, or may be 0.1 mm or more and 0.4 mm or less. A conductive material forming the coil electrode 22A and the lead wire 23 may be, for example, copper, silver, gold, platinum, and alloys thereof. In order to prevent a short circuit, the lead wire 23 may include an insulating coat made of fluoropolymer, for example. As shown in Figs. 2 and 3, a fixing means 24 for fixing the lead wire 23 may be provided. The fixing means 24 may be a joint member such as an adhesive, or may be a tube contracted on the inner shaft 32 as in the illustrated example.

The handle 40 is connected to the above-described catheter body 25 from the proximal side. The handle 40 is a part grasped by an operator (surgeon) during the use of the balloon catheter system 10. Thus, the handle 40 preferably has a design that allows an operator to easily grasp and operate the handle 40 by his/her hand. A material forming the handle 40 preferably has excellent chemical resistance. For example, polycarbonate or ABS resin can be used.

The handle 40 has therein the liquid flow path 28 leading to the lumen 31L of the outer shaft 31. The liquid flow path 28 is opened to the outside of the balloon catheter 20 at a liquid-flow-path opening 42 provided in the handle 40. As described below, a liquid flow member 70 of the liquid control system 50 is communicated with the liquid flow path 28 through the liquid-flow-path opening 42.

The handle 40 is fixed on the proximal end of the outer shaft 31. Although not shown, the handle is provided with a manipulator. The manipulator is connected to the proximal end of the inner shaft 32. By operating the manipulator, the inner shaft 32 can be relatively moved with respect to the outer shaft 31. By moving the inner shaft 32 with respect to the outer shaft 31, the balloon 35 can be tensed and loosened.

As shown, a wiring 96 is connected to the handle 40. The wiring 96 is electrically connected to the lead wire 23. The wiring 96 is electrically connected to a control device 95 described later. The counter electrode 97 is also electrically connected to the control device 95 through the wiring 96.

Next, the liquid control system 50 is described. The liquid control system 50 supplies a liquid to the balloon catheter 20 including the outer shaft 31 and the balloon 35 connected to the outer shat 31, and discharges the liquid from the balloon catheter 20. As shown in Fig. 1, the liquid control system 50 includes a syringe pump 60, a liquid flow member 70, a reservoir 80, and a liquid flow controller 90. The syringe pump 60 supplies a liquid to the balloon catheter 20. The syringe pump 60 can collect the liquid and gas discharged from the balloon catheter 20. As described later, the reservoir 80 can accommodate the gas and the liquid discharged from the balloon catheter 20. The liquid flow member 70 forms a liquid flow path between the balloon catheter 20 and the syringe pump 60. The liquid flow member 70 also forms a liquid flow path between the syringe pump 60 and the reservoir 80. The liquid flow controller 90 opens and closes the liquid flow paths formed by the liquid flow member 70. Whether the balloon catheter 20 or the reservoir 80 is communicated with the liquid control system 50 through the liquid flow member 70 can be selected by the liquid flow controller 90.

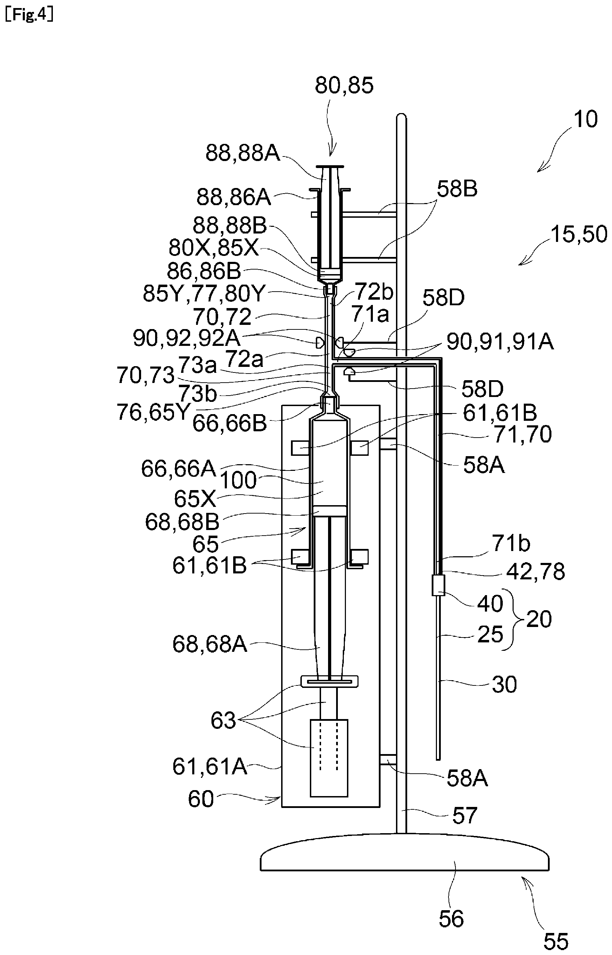

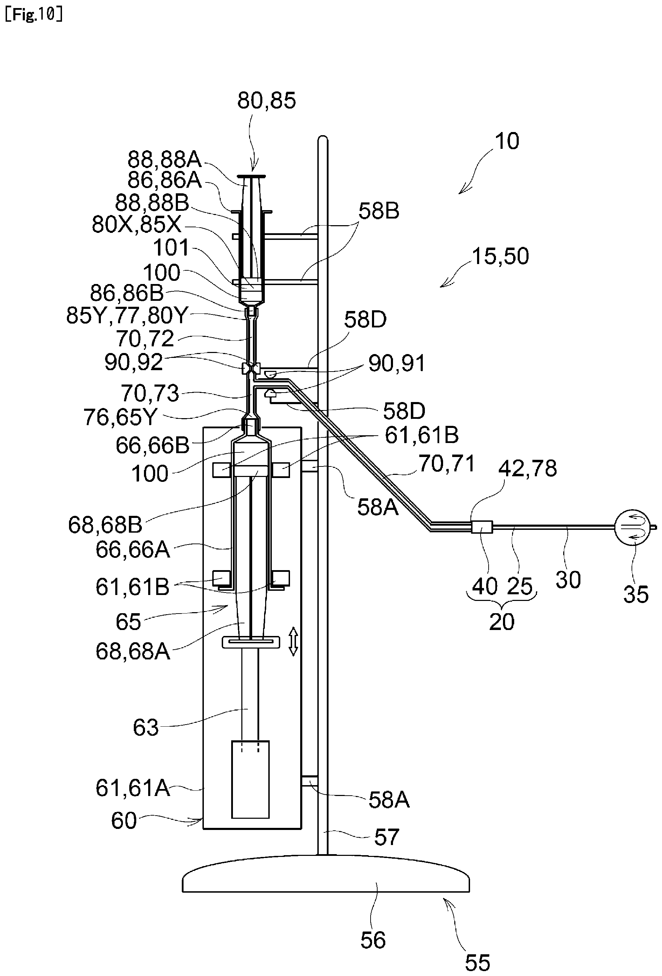

As shown in Figs. 4 to 10, the liquid control system 50 may further include a support 55 that holds the syringe pump 60 and the reservoir 80. The illustrated support 55 includes a base 56, a support rod 57, and holding parts 58A, 58B, 58D. The base 56 is positioned on a floor or the like. The base 56 has a flat shape. The support rod 57 extends upward from the base 56. The holding parts 58A, 58B, 58D extend horizontally from the support rod 57. Vertical positions of the holding parts 58A, 58B and directions in which the holding parts 58A, 58B, 58D extend from the support rod 57 may be adjustable. In the illustrated example, the first holding part 58A holds the syringe pump 60. The second holding part 85B holds the reservoir 80. The third holding part 58D holds the liquid flow controller 90.

The syringe pump 60 includes a syringe 65, a holding unit 61, and a drive 63. The syringe 65 accommodates a liquid 100 to be supplied to the balloon catheter 20. The syringe 65 accommodates the liquid 100 discharged from the balloon catheter 20.

The syringe 65 is not particularly limited. Various syringes can be applied to this embodiment. The illustrated syringe 65 includes a cylinder 66 and a piston (plunger) 68. The cylinder 66 includes a cylinder body 66A and a distal cylindrical part 66B. The cylinder body 66A is a cylindrical member. The distal cylindrical part 66B projects from the cylinder body 66A. The distal cylindrical part 66B forms a distal end opening 65Y of the syringe 65. A part of the piston 68 is inserted into the cylinder 66. The piston 68 is relatively movable with respect to the cylinder 66. The piston 68 can be moved to and from (reciprocated with respect to) the cylinder 66 along an axial direction of the syringe 65. Namely, the piston 68 can be moved close to the cylinder 66 along the axial direction of the syringe 65, and can be moved away from the cylinder 66 along the axial direction of the syringe 65. The piston 68 has a piston body 68A and a gasket 68B. The gasket 68B forms a distal end of the piston 68, and is inserted into the cylinder 66. The gasket 68B of the piston 68 and the cylinder 66 delimit an accommodation chamber 65X. As shown in Fig. 4, the liquid 100 can be accommodated in the accommodation chamber 65X.

The syringe 65 may be formed by a resin material suitable for enclosing the liquid 100. Polypropylene or polycarbonate is an example of the material of the cylinder 66 and the piston body 68A. The gasket 68B of the piston 68 may be formed of a material that has excellent liquid tightness with the cylinder 66. Butyl rubber is an example of the gasket 68B.

The holding unit 61 holds the syringe 65. The holding unit 61 holds only one of the cylinder 66 and the piston 68 of the syringe 65. The holding unit 61 does not hold the other of the cylinder 66 and the piston 68 of the syringe 65. Thus, in the syringe 65 held by the holding unit 61, the piston 68 and the cylinder 66 can be relatively moved to each other. From a hygiene viewpoint, the syringe 65 is preferably disposable. In order that the drive 63 and so on can be repeatedly used while the syringe 65 is disposable, the holding unit 61 may detachably hold the syringe 65. The holding unit 61 may be capable of holding syringes 65 of different sizes.

For example, the illustrated holding unit 61 includes a holding base 61A and a clamp 61B. The illustrated holding unit 61 is configured to hold the syringe 65 in a predetermined orientation. Specifically, the holding unit 61 holds the syringe 65 such that the liquid flow member 70 is connected to the syringe 65 vertically from above. The holding unit 61 holds the syringe 65 such that the cylinder 66 is positioned vertically above the piston 68. The distal end opening 65Y of the syringe 65 is opened vertically upward. In the illustrated example, in the accommodation chamber 65X, gas in the accommodation chamber 65X is positioned in an area adjacent to the distal end opening 65Y. Namely, when the gas and the liquid are included in the accommodation chamber 65X, the piston 68 pushed into the cylinder 66 first discharges the gas from the syringe 65, and then discharges the liquid from the syringe 65.

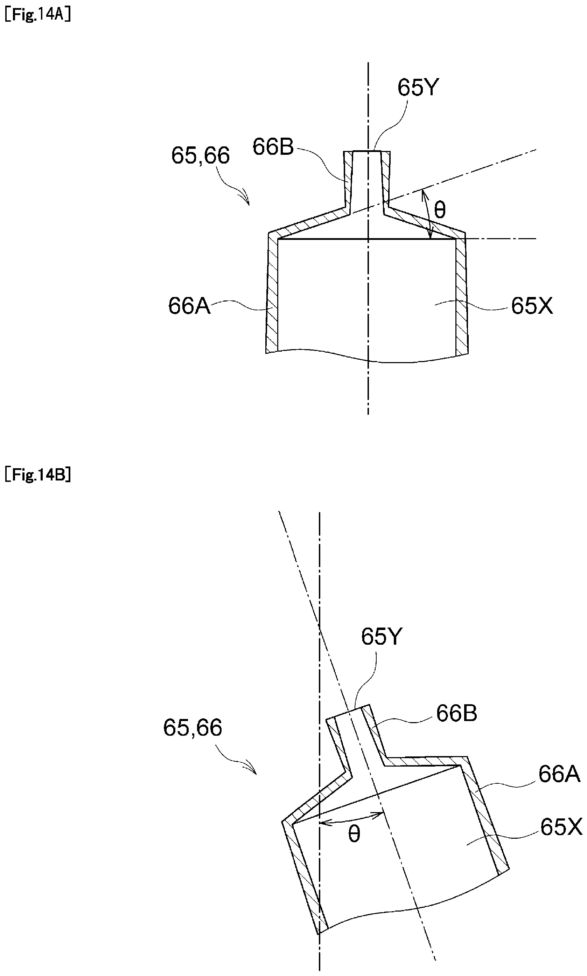

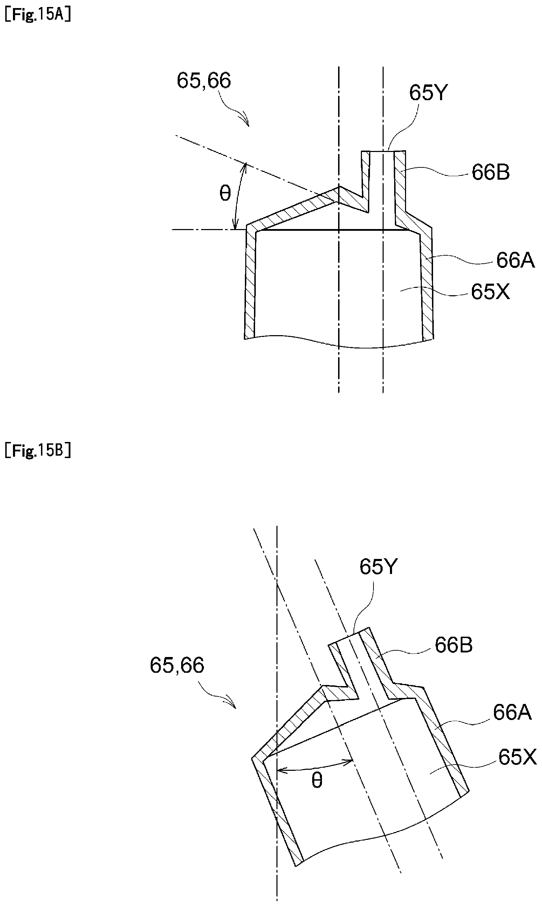

As shown in Fig. 14A, when a distal end surface of the cylinder has an inclination, the cylinder 66 may be inclined as shown in Fig. 14B, as long as the inclination does not exceed an inclination angle θ. Even when the cylinder 66 is supported at an inclination, the gas is first discharged and then the liquid is discharged, as described above. As shown in Fig. 15A, a syringe 65 of an eccentric type may be used. In the syringe 65 of the eccentric type, the distal end opening 65Y is not aligned with a center axis of the cylinder 66 but deviates from the center axis of the cylinder 66. The syringe 65 shown in Fig. 15A may also be inclined as shown in Fig. 15B, as long as the inclination does not exceed an inclination angle θ of a distal end surface of the cylinder 66. Also in the example shown in Fig. 15B, the gas is first discharged and then the liquid is discharged.

The drive 63 relatively moves the piston 68 with respect to the cylinder 66. In the illustrated example, the holding unit 61 holds the cylinder 66 of the syringe 65. In the illustrated example, the drive 63 drives the piston 68 of the syringe 65. The drive 63 moves the piston 68 with respect to the cylinder 66 held by the holding unit 61. The drive 63 may move the piston to and from the cylinder. In the illustrated example, the drive 63 relatively moves the piston 68 vertically upward and downward with respect to the cylinder 66. Particularly in the illustrated example, the piston 68 is relatively moved vertically with respect to the cylinder 66. The drive 63 may adjust a relative movement amount of the piston 68 and the cylinder 66. The drive 63 may adjust an amplitude in the to-and-fro movement of the piston 68. The drive 63 may adjust a speed of the to-and-fro movement of the piston 68. For example, the drive 63 may allow a relative movement speed at which the piston 68 is pushed into the cylinder 66 and a relative movement speed at which the piston 68 is drawn out from the cylinder 66 to be different from each other.

In the illustrated example, the holding unit 61 holds the cylinder 66, and the drive 63 moves the piston 68. However, not limited thereto, the the holding unit 61 may hold the piston 68, and the drive 63 may move the cylinder 66.

The drive 63 may be composed of various drive units. The drive 63 may have a linear motor. The drive 63 may have a motor, and a mechanism for converting rotational motion to linear motion. The motor may be a DC motor, an AC motor, a stepper motor, or a servo motor. The mechanism for converting rotational motion to linear motion is not particularly limited. This mechanism may be a ball screw mechanism, or a mechanism with a connecting rod and a crankshaft.

Next, the reservoir 80 is described. The reservoir 80 is coupled to the syringe 65 of the syringe pump 60 by the liquid flow member 70. The reservoir 80 has a reservoir accommodation chamber 80X. The reservoir accommodation chamber 80X can accommodate a liquid. The reservoir accommodation chamber 80X is communicated with the accommodation chamber 65X of the syringe 65 through the liquid flow member 70. The reservoir accommodation chamber 80X has a reservoir opening 80Y that opens the reservoir accommodation chamber 80X.

The liquid flow member 70 is communicated with the reservoir accommodation chamber 80X through the reservoir opening 80Y. The liquid flow member 70 is connected to the reservoir 80 vertically from below. The reservoir opening 80Y of the reservoir 80 is opened vertically downward. This example allows the reservoir 80 to stably collect the gas moving in the liquid flow member 70. According to the illustrated example, in the reservoir accommodation chamber 80X, the gas is positioned in an area away from the reservoir opening 80Y. In the reservoir accommodation chamber 80X, the liquid in the reservoir accommodation chamber 80X is positioned in an area adjacent to the reservoir opening 80Y. Thus, the reservoir 80 releases the liquid, prior to the gas, to the liquid flow member 70. Namely, when the reservoir accommodation chamber 80X contains the gas and the liquid, the liquid is first discharged from the reservoir 80, and then the gas is discharged from the reservoir 80.

The reservoir 80 may be formed by a resin material suitable for enclosing the liquid 100. Polypropylene or polycarbonate is an example of the material of the reservoir 80.

In the illustrated example, the reservoir 80 includes a reservoir syringe 85. The reservoir syringe 85 may have the same structure as the aforementioned syringe 65 of the syringe pump 60. The reservoir syringe 85 includes a syringe 86 connected to the liquid flow member 70, and a piston 88 that is relatively movable with respect to the cylinder 86. The cylinder 86 includes a cylinder body 86A and a distal cylindrical part 86B. The cylinder body 86A is a cylindrical member. The distal cylindrical part 86B projects from the cylinder body 86A. The distal cylindrical part 86B forms a distal end opening 85Y of the reservoir syringe 85. The distal end opening 85Y of the reservoir syringe 85 forms the reservoir opening 80Y of the reservoir 80. A part of the piston 88 is inserted into the cylinder 86. The piston 88 is relatively movable with respect to the cylinder 86. The piston 88 can be moved to and from the cylinder 86 along an axial direction of the reservoir syringe 85. Namely, the piston 88 can be moved close to the cylinder 86 along the axial direction of the reservoir syringe 85, and can be moved away from the cylinder 86 along the axial direction of the reservoir syringe 85.

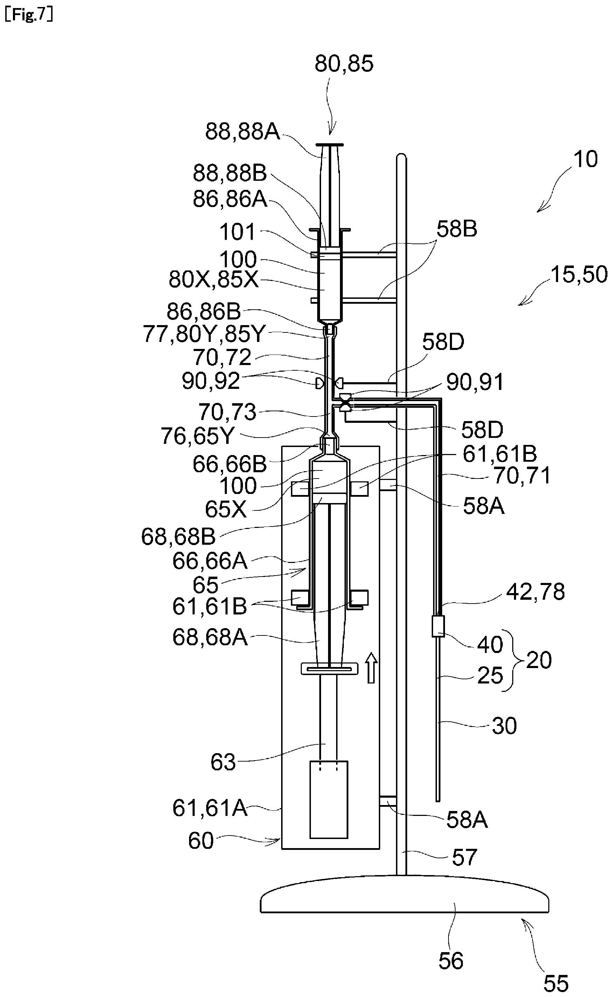

The piston 88 has a piston body 88A and a gasket 88B. The gasket 88B forms a distal end of the piston 88, and is inserted into the cylinder 86. The gasket 88B of the piston 88 and the cylinder 86 delimit an accommodation chamber 85X. The accommodation chamber 85X of the reservoir syringe 85 forms the reservoir accommodation chamber 80X of the reservoir 80. As shown in Fig. 7, the liquid 100 can be accommodated in the accommodation chamber 85X.

The reservoir syringe 85 may be formed by the same material as the aforementioned syringe 65.

The cylinder 86 of the reservoir syringe 85 is positioned vertically below the piston 88 of the reservoir syringe 85. The piston 88 of the reservoir syringe 85 is relatively moved upward and downward with respect to the cylinder 86 of the reservoir syringe 85. The distal end opening 85Y of the reservoir syringe 85 is opened vertically downward. The liquid flow member 70 is connected to the reservoir syringe 85 vertically from below. As shown in Fig. 7, the gas 101 in the accommodation chamber 85X is positioned in an area away from the distal end opening 85Y. The liquid 100 in the accommodation chamber 85X is positioned in an area adjacent to the distal end opening 85Y. Namely, when the accommodation chamber 85X contains the gas and the liquid, the liquid 100 first flows out from the accommodation chamber 85X to the liquid flow member 70. The gas 101 is not discharged from the accommodation chamber 85X untill all of the liquid 100 has flowed out from the accommodation camber 85X.

When the reservoir syringe 85 is used and the gas or the liquid is supplied from the liquid flow member 70 to the reservoir 80, the piston 88 can be relatively moved with respect to the cylinder 86 to enlarge the accommodation chamber 85X. When the gas or the liquid in the reservoir 80 is suctioned to be discharged to the liquid flow member 70, the piston 88 can be relatively moved with respect to the cylinder 86 to narrow the accommodation chamber 85X.

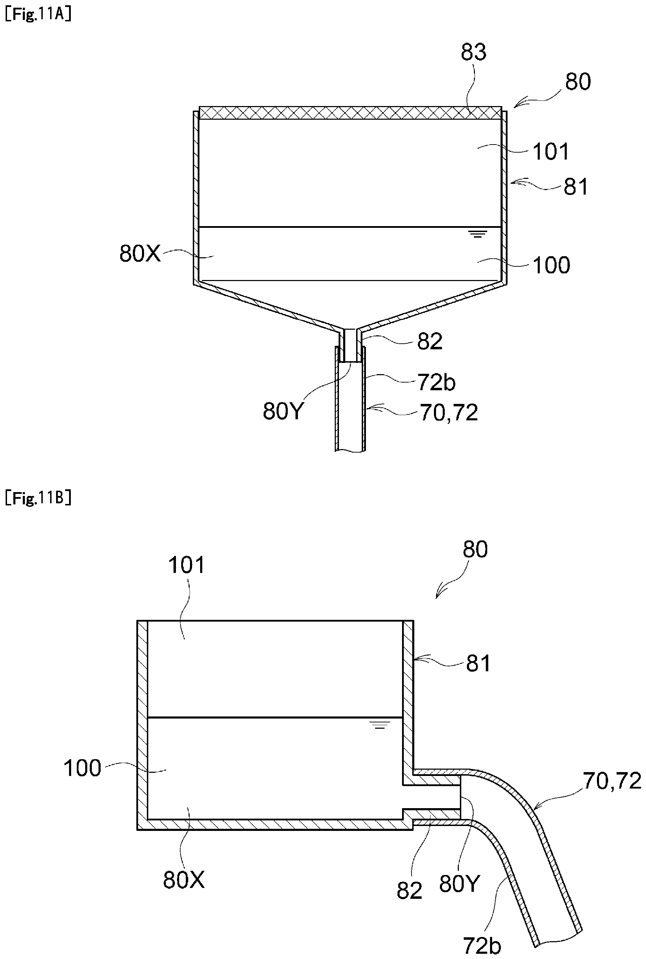

The reservoir 80 is not limited to the illustrated reservoir syringe 85. As in an example shown in Fig. 11A, the reservoir 80 may include a container 81. The illustrated container 81 includes a lower cylindrical part 82 projecting vertically downward. The lower cylindrical part 82 forms the reservoir opening 80Y. The liquid flow member 70 is connected to the lower cylindrical part 82 from below. A bottom surface of the container 81, where the lower cylindrical part 82 is provided, is positioned at the lowest point. Such a container 81 can also discharge the liquid 100 prior to the gas 101. The reservoir 80 shown in Fig. 11A may be provided with a lid member 83 in an upper opening of the container 81. The lid member 83 can reduce entering of foreign matter into the reservoir 80. In order to smoothen supply of the liquid 100 and the gas 101 to the container 81 and discharge of them therefrom, at least one of the container 81 and the lid member 83 may be breathable. The lid member 83 shown in Fig. 11A may be a filter member.

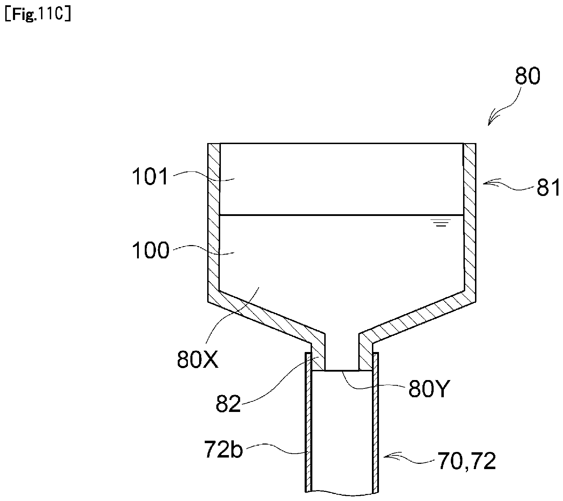

As shown in Fig. 11B, the reservoir opening 80Y may be located at a lower position of a side surface of the reservoir 80. However, as shown in Fig. 11C, the reservoir opening 80Y located centrally in the bottom surface of the reservoir is preferred, because it allows the liquid in the reservoir 80 to be discharged without leaving any liquid. In this example, the reservoir opening 80Y extends vertically.

Next, the liquid flow member 70 is described. The liquid flow member 70 couples the syringe 65 of the syringe pump 60 and the balloon catheter 20. Namely, the liquid flow member 70 extends between the syringe 65 of the syringe pump 60 and the balloon catheter 20. A liquid can flow through the liquid flow member 70 between the syringe 65 of the syringe pump 60 and the balloon catheter 20. The liquid flow member 70 couples the syringe 65 of the syringe pump 60 and the reservoir 80. The liquid flow member 70 extends between the syringe 65 of the syringe pump 60 and the reservoir 80. A liquid can flow through the liquid flow member 70 between the syringe 65 of the syringe pump 60 and the reservoir 80. The liquid flow member 70 may include a tube to enable transfer of the liquid and the gas.

The liquid flow member 70 may be formed of a flexible material with excellent chemical resistance. Examples of the material used for the liquid flow member 70 include fluoropolymers, polyamides, polyurethane-based polymers, polyimides or the like.

As shown in Fig. 4, the liquid flow member 70 may include a first line part 71 connected to the balloon catheter 20, and a second line part 72 connected to the reservoir 80. The first line part 71 and the second line part 72 may be soft tubes. The liquid flow member 70 including the second line part 72 is connected to the cylinder 66 of the syringe pump 60 vertically from above. The tubular liquid flow member 70 may be connected to the cylinder 66 by inserting thereinto the distal cylindrical part 66B of the cylinder 66. The second line part 72 is connected to the reservoir 80 vertically from below. The tubular second line part 72 may be connected to the reservoir 80 by inserting thereinto the distal cylindrical part 86B of the reservoir 80 or the lower cylindrical part 82. The first line part 71 is connected to the balloon catheter 20 vertically from above.

In the example shown in Fig. 4, the liquid flow member 70 further has a joint line part 73. The joint line part 73 is a tubular portion including a first end 73a and a second end 73b. The first line part 71 is a tubular portion including a first end 71a and a second end 71b. The second line part 72 is a tubual portion including a first end 72a and a second end 72b. The first end 71a of the first line part 17, the first end 72a of the second line part 72, and the first end 73a of the joint line part 73 join each other. The second end 71b of the first line part 71 is connected to the balloon catheter 20. The second end 72b of the second line part 72 is connected to the reservoir 80 vertically from below. The second end 73b of the joint line part 73 is connected to the syringe 65 vertically from above. The first line part 71 may be positioned gradually vertically downward from the first end 71a toward the second end 71b. The second line part 72 may be positioned gradually vertically upward from the first end 72a toward the second end 72b. The joint line part 73 may be positioned gradually vertically downward from the first end 73a toward the second end 73b. As shown in Fig. 4, the first line part 71, the second line part 72, and the joint line part 73 may be integrally formed seamlessly.

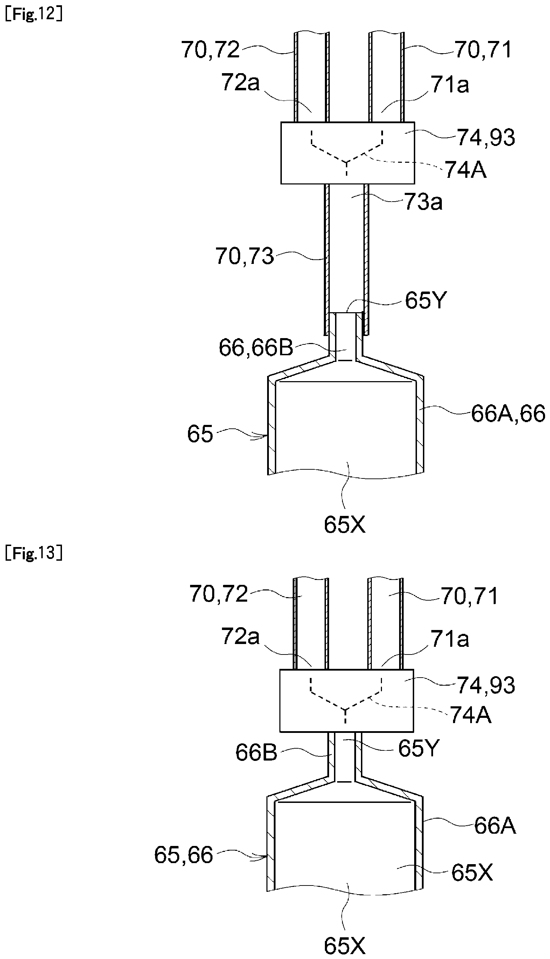

Note that the structure of the liquid flow member 70 is not limited to the example shown in Fig. 4. As shown in Fig. 12. the first end 71a of the first line part 71, the first end 72a of the second line part 72, and the first end 73a of the joint line part 73 may be connected to each other through a liquid flow connector 74. In the example shown in Fig. 12, an interior of the first line part 71, an interior of the second line part 72, and an interior of the joint line part 73 are communicated with each other through an interior path 74A of the liquid flow connector 74.

In an example shown in Fig. 13, the liquid flow member 70 does not include the joint line part 73. The liquid flow member 70 includes the first line part 71, the second line part 72, and the liquid flow connector 74. The liquid flow connector 74 is attached to the distal cylindrical part 66B of the syringe 65. The first end 71a of the first line part 71 and the first end 72a of the second line part 72 are also attached to the liquid flow connector 74. The interior of the first line part 71 and the interior of the second line part 72 are communicated with the accommodation chamber 65X of the syringe 65 through the interior path 74A of the liquid flow connector 74.

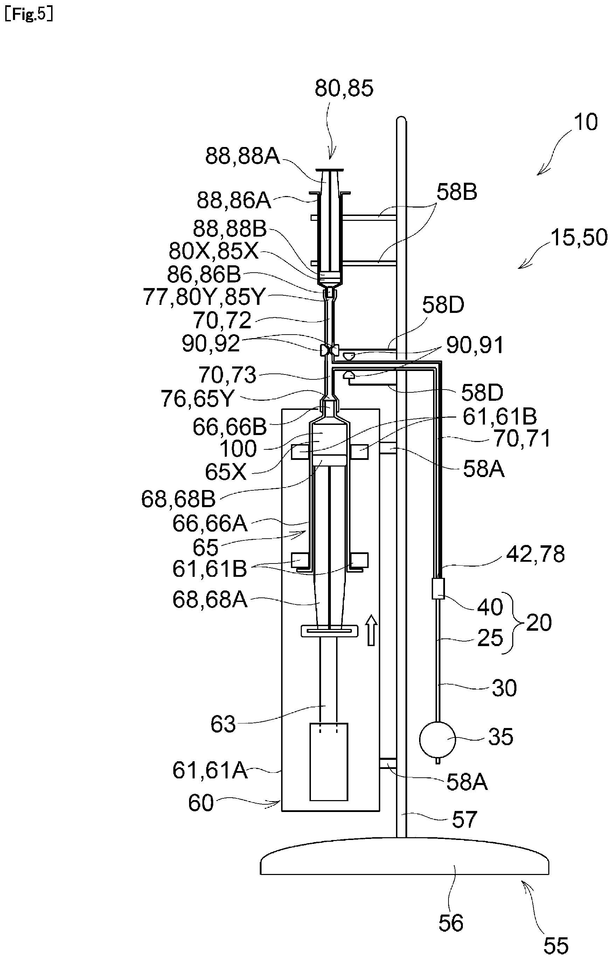

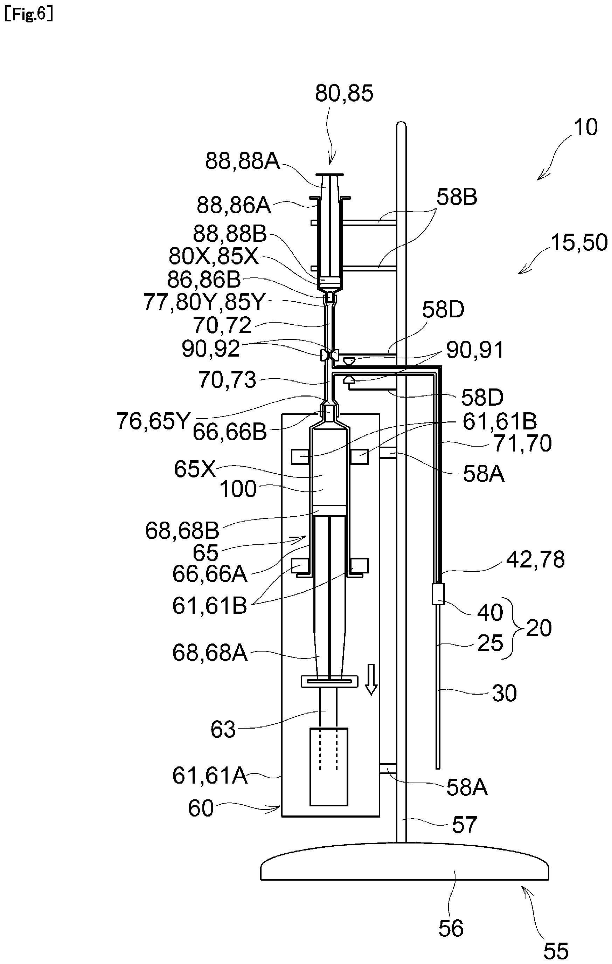

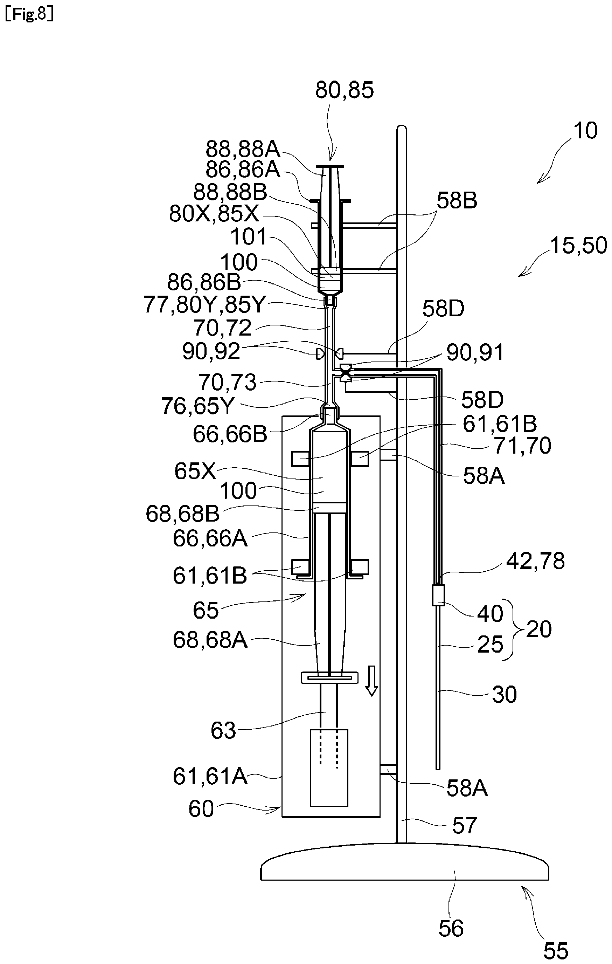

Next, the liquid flow controller 90 is described. The liquid flow controller 90 controls flow of the liquid in the liquid flow member 70. The liquid flow controller 90 switches between a first state shown in Figs. 5 and 6, and a second state shown in Figs. 7 and 8. In the first state, the communication between the syringe 65 and the reservoir 80 through the second line part 72 is blocked, and the communication between the syringe 65 and the balloon catheter 20 through the first line part 71 is allowed (obtained). In the second state, the communication between the syringe 65 and the balloon catheter 20 through the first line part 71 is blocked, and the communication between the syringe 65 and the reservoir 80 through the second line part 72 is allowed (obtained).

The liquid flow controller 90 may include a pinch valve. The pinch valve pushes a tube forming a liquid flow path from outside to close a flow path. When closing the flow path, the pinch valve is not in contact with the liquid flowing through the liquid flow path. The pinch valve can avoid contamination of the liquid. The operation of the pinch valve can be realized using a solenoid. Thus, the opening and closing of the flow path can also be switched quickly.

In the example shown in Fig. 4, the liquid flow controller 90 includes a first pinch valve 91 that opens and closes the first line part 71, and a second pinch valve 92 that opens and closes the second line part 72. In the example shown in Fig. 4, by closing the second pinch valve 92 and keeping the first pinch valve 91 open, the first state is achieved. By closing the first pinch valve 91 and keeping the second pinch valve 92 open, the second state is achieved.

Differently from the example shown in Fig. 4, the liquid flow controller 90 may include a three-way valve 93. In the aforementioned example shown in Figs. 12 and 13, the liquid flow connector 74 may be the three-way valve 93. In this example, the first line part 71, the second line part 72, and the joint line part 73 are connected to each other by the three-way valve 93. By switching the three-way valve 93, the flow path connected to the joint line part 73 can be switched between the first line part 71 and the second line part 72.

As described above, the liquid control system 50 shown in Fig. 4 includes the support 55 that supports the syringe pump 60 and the reservoir 80. As shown in Fig. 4, the support 55 also supports the balloon catheter 20 before it is inserted into a body of a patient. The support 55 may support the liquid flow member 70 in order to adjust directions in which the liquid flow member 70 is connected to the other constituent elements 20, 60, 80. The use of the support 55 can make stable a posture of the syringe 65 such that the distal end opening 65Y of the syringe 65 is opened vertically upward. The use of the support 55 can make stable a posture of the reservoir 80 such that the reservoir opening 80Y of the reservoir 80 is opened vertically downward.

Further in the example shown in Fig. 4, a pump connection position 76 at which the liquid flow member 70 and the syringe pump 60 (syringe 65) held by the support 55 are connected to each other is positioned vertically above a catheter connection position 78 at which the liquid flow member 70 and the balloon catheter 20 held by the support 55 are connected to each other. A reservoir connection position 77 at which the liquid flow member 70 and the reservoir 80 held by the support 55 is positioned vertically above the pump connection position 76 at which the liquid flow member 70 and the syringe pump 60 (syringe 65) held by the support 55 are connected to each other. Such positioning by the support 55 allows a preparatory work prior to use of the balloon catheter system 10 to be performed stably and properly, as described later.

As shown in Fig. 1, the balloon catheter system 10 and the liquid control system 50 further include the control device 95. The illustrated control device 95 includes a power control unit 95A, a liquid control unit 95B, a storage unit 95C, and an input unit 95D. The control device 95 may be a general computer including a processor, a memory, etc. The computer forming the control device 95 may be a desktop or notebook personal computer (PC), or a portable information terminal such as a tablet. The power control unit 95A and the liquid control unit 95B may be processors. The processor may be a central processing unit (CPU). The processor may be a micro processing unit (MPU), graphical processing unit (GPU), field-programmable gate array (FPGA), etc. The power control unit 95A and the liquid control unit 95B may be software instead of or in addition to hardware such as a CPU. The memory unit 95C may be any storage medium, such as a an optical disk such as a hard disk or a memory. The input unit 95D may include a mouse, keyboard, touch panel, etc. The control device 95 may further include a display unit such as a display.

As described above, the counter electrode 97 is electrically connected to the control device 95 through the wiring 96. The heating member 22 of the balloon catheter 20 is electrically connected to the control device 95 through the lead wire 23 and the wiring 96. The power control unit 95A controls the high-frequency current conduction to the counter electrode 97 and the heating member 22, based on information stored in the storage unit 95C and information inputted through the input unit 95D. A temperature sensor may be provided in the balloon 35 of the balloon catheter 20. The power control unit 95A may control the high-frequency current conduction to the counter electrode 97 and the heating member 22, based on information from the temperature sensor.

As shown in Fig. 1, the syringe pump 60 and the liquid flow controller 90 are electrically connected to the control device 95 through the wiring 96. The liquid control unit 95B controls operations of the syringe pump 60 and the drive 63, based on information stored in the storage unit 95C and information inputted through the input unit 95D. The liquid control unit 95B controls an operation of the liquid flow controller 90, based on information stored in the storage unit 95C and information inputted through the input unit 95D.

An example of a using method of the balloon catheter system 10 as structured above is described.

The balloon catheter 20 included in the balloon catheter system 10, the syringe 65, the liquid flow member 70, and the reservoir 80 form the balloon catheter assembly 15. The balloon catheter assembly 15 may be distributed or otherwise handled as a single unit. In the balloon catheter system 10, the balloon catheter assembly 15 is a constituent element that is in contact with a liquid. The disposable balloon catheter assembly 15 is desired from a hygiene viewpoint. In the balloon catheter assembly 15 prior to use, a liquid 100 an amount of which is needed for treatment may be accommodated in the accommodation chamber 65X of the syringe 65.

As shown in Fig. 4, the balloon catheter assembly 15 is attached to the support 55. Before the balloon catheter assembly 15 is attached to the support 55, a constituent element of the balloon catheter system 10, which is not included in the balloon catheter assembly 15, may be attached to the support 55. For example, the holding unit 61 and the drive 63 of the syringe pump 60 are held by the first holding part 58A. The liquid flow controller 90 is held by the third holding part 58D.

The syringe 65 of the balloon catheter assembly 15 is attached to the holding unit 61. The syringe 65 is attached to the holding unit 61 such that the distal end opening 65Y formed by the cylinder 66 is opened vertically upward. The cylinder 66 of the syringe 65 held by the holding unit 61 is positioned vertically above the piston 68. In the specific example shown in Fig. 4, the syringe 65 is held by the holding unit 61 such that its center axis is in parallel with the vertical direction. In this example, the piston 68 is relatively movable with respect to the cylinder 66 in a direction in parallel with the vertical direction.

The reservoir 80 is attached to the second holding part 58B of the support 55. The reservoir 80 is attached to the second holding part 58B such that the reservoir opening 80Y is opened vertically downward. In the example shown in Fig. 4, the reservoir 80 is the reservoir syringe 85. The cylinder 86 of the reservoir syringe 85 is attached to the second holding part 58B. In the reservoir syringe 85 held by the support 55, the piston 88 is relatively movable with respect to the cylinder 86 held by the first holding part 58A. The cylinder 86 held by the second holding part 58B is positioned vertically below the piston 88. In the specific example shown in Fig. 4, the reservoir syringe 85 is held by the second holding part 58B such that its center axis is in parallel with the vertical direction. In this example, the piston 88 is relatively movable with respect to the cylinder 86 in a direction in parallel with the vertical direction.

The balloon catheter 20 may be held by a user or the like, or may be held by a specialized jig. The handle 40 of the balloon catheter 20 may be held such that the catheter body 25 hangs down from the handle 40.

The illustrated liquid flow member 70 includes the first line part 71 and the second line part 72. The first line part 71 is positioned to extend between a pair of blocks 91A of the first pinch valve 91 included in the liquid flow controller 90. The second line part 72 is positioned to extend between a pair of blocks 92A of the second pinch valve 92 included in the liquid flow controller 90. As shown in Fig. 4, the joint line part 73 of the liquid flow member 70 is connected, at its second end 73b, to the syringe 65 vertically from above. The second line part 72 of the liquid flow member 70 is connected, at its second end 72b, to the reservoir 80 vertically from below. The first line part 71 is connected, at its second end 71b, to the balloon catheter 20 vertically from above.

Next, the gas is discharged from the balloon catheter 20 in the procedure shown in Figs. 5 to 8. In the step of discharging gas (gas discharging step) shown in Figs. 5 to 8, a relative position of the outer shaft 31 and the inner shaft 32 may be adjusted to a position at which the balloon 35 can be inflated.

First, a first step (supply step) shown in Fig. 5 is performed In the first step, the liquid flow member 70 is placed in the first state. In the first state, the communication between the syringe 65 and the reservoir 80 is blocked, and the communication between the syringe 65 and the balloon catheter 20 is allowed. Not only in this step but also in steps described later, change of the state of the liquid flow member 70 is realized by the liquid flow controller 90 that is operated based on a control signal from the liquid control unit 95B of the control device 95. As shown in Fig. 5, the pair of block 91A forming the first pinch valve 91 are separated from each other. The inside flow path in the first line part 71 extending between the pair of first blocks 91A is thus opened. The pair of blocks 92A forming the second pinch valve 92 are close to each other. The inside flow path in the second line part 72 extending between the pair of blocks 92A is thus closed.

In the first step shown in Fig. 5, the liquid flow member 70 is maintained in the first state, and the piston 68 of the syringe 65 is pushed into the cylinder 66. When the piston 68 moves into the cylinder 66, the liquid 100 is ejected from the syringe pump 60. Namely, the liquid 100 accommodated in the accommodation chamber 65X is ejected from the distal end opening 65Y into the liquid flow member 70. The liquid 100 is supplied to the liquid flow path 28 of the balloon catheter 20 through the liquid flow member 70 in the fist state. The liquid 100 supplied to the balloon catheter 20 passes through the lumen 31L of the outer shaft 31 to reach the balloon 35. As shown in Fig. 5, the supply of the liquid 100 from the syringe 65 to the shaft member 30 may be performed until the balloon 35 is inflated.

Not only in the first step but also in the subsequent steps, the operation of the syringe 65 is caused by the drive 63. The drive 63 is operated by an electric signal from the liquid control unit 95B of the control device 95. The drive 63 moves the piston 68 with respect to the cylinder 66 held by the holding unit 61. The movement length and the movement speed of the piston 68 are controlled by the liquid control unit 95B of the control device 95.

Next, a second step (discharge step) shown in Fig. 6 is performed. In the second step, the piston 68 is drawn out from the cylinder 66, while the liquid flow member 70 is maintained in the first state. When the piston 68 is drawn out from the cylinder 66, the liquid 100 is suctioned to the syringe pump 60. The liquid 100 in the balloon catheter 20 is discharged from the balloon catheter 20 to move into the liquid flow member 70. Along with the discharge of the liquid 100 from the balloon catheter 20, the balloon 35 is deflated. The liquid 100 in the liquid flow member 70 is suctioned from the liquid flow member 70 into the accommodation chamber 65X of the syringe 65.

In the second step, the gas (bubbles) remaining in the balloon catheter 20 is moved, together with the liquid 100, from the liquid flow path 28 of the balloon catheter 20 into the liquid flow member 70. As shown in Fig. 6, the balloon catheter 20 is held to hang down from the catheter connection position 78 at which the balloon catheter 20 and the liquid flow member 70 are connected to each other. Thus, the gas remaining in the balloon catheter 20 is smoothly moved, together with the liquid 100, into the liquid flow member 70. The gas and the liquid 100 in the liquid flow member 70 are suctioned by the syringe pump 60 toward the syringe 65. This step allows the gas and the liquid 100 to be collected in the accommodation chamber 65X of the syringe 65.

Next, a third step (transport step) shown in Fig. 7 is performed. In the third step, the liquid flow member 70 is placed in the second state. In the second state, the communication between the syringe 65 and the balloon catheter 20 is blocked, and the communication between the syringe 65 and the reservoir 80 is allowed. As described above, change of the state of the liquid flow member 70 is realized based on a control signal from the liquid control unit 95B of the control device 95. As shown in Fig. 7, the pair of blocks 91A forming the first pinch valve 91 are close to each other. The inside flow path in the first line part 71 extending between the pair of first blocks 91A is thus closed. The pair of blocks 92A forming the second pinch valve 92 are separated from each other. The inside flow path in the second line part 72 extending between the pair of blocks 92A is thus opened.

In the third step shown in Fig. 7, the liquid flow member 70 is maintained in the second state, and the piston 68 of the syringe 65 is pushed into the cylinder 66. When the piston 68 is moved into the cylinder 66, the gas 101 and the liquid 100 in the accommodation chamber 65X are ejected from the syringe pump 60. The liquid flow member 70 is connected to the syringe 65 vertically from above. The syringe 65 is located such that the distal end opening 65Y is opened vertically upward. Thus, when the piston 68 moves into the cylinder 66, the gas 101 is ejected prior to the liquid 100. Namely, the gas 101 can be stably discharged from the accommodation chamber 65X of the syringe 65. The gas 101 and the liquid 100 discharged from the syringe pump 60 are moved toward the reservoir 80.

In the illustrated example, the reservoir 80 is the reservoir syringe 85. The reservoir syringe 85 forming the reservoir 80 is held by the second holding part 58B of the support 55 such that the cylinder 86 and the piston 88 are relatively movable to each other. In the illustrated example, the second holding part 58B holds only the cylinder 86. Thus, the piston 88 is relatively moved with respect to the cylinder 86 in accordance with volumes of the gas 101 and the liquid 100 flowing into the accommodation chamber 85X of the reservoir syringe 85. In this manner, the accommodation chamber 85X has a volume capable of accommodating the gas 101 and the liquid 100 pumped thereinto.

The liquid flow member 70 is connected to the reservoir 80 vertically from below. The reservoir 80 is located such that the reservoir opening 80Y is opened vertically downward. In the illustrated example, the reservoir 80 is the reservoir syringe 85. The liquid flow member 70 is connected to the reservoir syringe 85 vertically from below. The reservoir syringe 85 is located such that the distal end opening 85Y is opened vertically downward. Thus, the gas 101 is pumped, prior to the liquid 100, to into the reservoir 80. In the accommodation chamber 85X of the reservoir syringe 85, the gas 101 is positioned vertically above the liquid 100. In the accommodation chamber 85X, the liquid 100 is positioned in a vertically lower area adjacent to the distal end opening 85Y.

The third step may be performed until not only the gas 101 but also the liquid 100 is accommodated in the reservoir 80. This example allows the gas to be more reliably discharged from the liquid flow member 70 and the syringe 65 and to be collected in the reservoir 80.

In this manner, the gas 101 discharged from the balloon catheter 20 can be collected in the reservoir 80 stably and smoothly. Along with the discharge of the gas from the balloon catheter 20, the gas 101 can also be discharged from the liquid flow member 70 that is a liquid flow path between the syringe pump 60 and the balloon catheter 20. Namely, it is not necessary to perform discharge of the gas from the liquid flow path between the syringe pump 60 and the balloon catheter 20, separately from discharge of the gas from the balloon catheter 20. This can simplify the preparation before use of the balloon catheter system 10.

In the illustrated example, the pump connection position 76 at which the liquid flow member 70 and the syringe pump 60 are connected to each other is positioned vertically above the catheter connection position 78 at which the liquid flow member 70 and the balloon catheter 20 are connected to each other. This specific example allows the gas discharged from the balloon catheter 20 to be stably guided to the syringe 65.

Similarly, the reservoir connection position 77 at which the liquid flow member 70 and the reservoir 80 are connected to each other is positioned vertically above the pump connection position 76 at which the liquid flow member 70 and the syringe pump 60 are connected to each other. This specific example allows the gas in the syringe 65 and in the liquid flow member 70 to be stably guided to the reservoir 80, when the liquid is pumped from the syringe 65 to the reservoir 80.

In the illustrated example, the liquid flow member 70 includes the first line part 71 connected to the balloon catheter 20, the second line part 72 connected to the reservoir 80, and the joint line part 73 connected to the syringe 65. The first line part 71 and the second line part 72 branch off from the joint line part 73. The lowermost portion (first end 72a) of the second line part 72 is located at the same vertical position as the uppermost portion (first end 73a) of the joint line part 73, or at a position vertically above the uppermost portion (first end 73a) of the joint line part 73. Thus, the gas ejected from the syringe 65 can be collected in the reservoir 80 stably and smoothly. Remaining of the gas in the joint line part 73 can be effectively avoided.

Similarly, in the illustrated example, the liquid flow path 70 includes the first line part 71 connected to the balloon catheter 20, the second line part 72 connected to the reservoir 80, and the joint line part 73 connected to the syringe 65. The first line part 71 and the second line part 72 branch off from the joint line part 73. The lowermost portion (first end 72a) of the second line part 72 is located at the same vertical position as the uppermost portion (first end 71a) of the first line part 71, or at a position vertically above the uppermost portion (first end 71a) of the first line part 71. Thus, the gas discharged from the balloon catheter 20 can be collected in the reservoir 80 stably and smoothly. Remaining of the gas in the first line part 71 can be effectively avoided.

In the conventional balloon catheter system, a surgeon or a technician manually operates the valve and the syringe and further adjusts an orientation and a position of the syringe to release the gas collected in the syringe outside a system composed of the syringe and the liquid flow path. In this point, in the aforementioned embodiment, the liquid flow member 70 is connected to the syringe 65 from above, and is connected to the liquid reservoir 80 from below. Thus, the gas collected in the syringe 65 can be sent to the liquid reservoir 80 easily and stably. In addition, the syringe 65 can be held in a predetermined posture by the syringe pump 60, and the piston 68 can be accurately relatively moved with respect to the cylinder 66 at a desired amount. These features greatly reduce the burden of preparatory work before use of the balloon catheter system 10.

The aforementioned method of discharging the gas includes the first step, the second step, and the third step. The first step, the second step, and the third step may be repeated multiple times in this order. This example allows the gas to be accurately discharged from the balloon catheter 20, and allows the gas to be accurately discharged from a portion of the liquid flow member 70, which serves as a liquid flow path to the balloon catheter 20.

As described above, according to the aforementioned embodiment, the gas 101 collected in the syringe 65 can be discharged to the reservoir 80 easily and stably. The effect of reducing the burden of preparatory work becomes more significant by repeating the first step, the second step, and the third step.

As a further modification example of the method of discharging the gas, the method of discharging the gas may include a fourth step (second discharge step), in addition to the first step, the second step, and the third step. The fourth step is performed after the third step. As shown in Fig. 8, in the fourth step, the liquid flow member 70 is placed in the second state, the liquid 100 is collected in the cylinder 66 by drawing out the piston 68 from the cylinder 66, with the gas remaining in the reservoir 80. As shown in Figs. 7 and 8, in the reservoir 80, the gas is positioned in an vertically upper area. In the reservoir 80, the liquid 100 is positioned in an vertically lower area adjacent to the reservoir opening 80Y. Thus, when the piston 68 is drawn out from the cylinder 66, only the liquid 100 can be discharged from the reservoir accommodation chamber 80X to be collected in the syringe 65, with the gas remaining in the reservoir accommodation chamber 85X of the reservoir 80. The liquid collected in the syringe 65 can be supplied to the balloon catheter 20. The fourth step can improve utilization efficiency of the liquid 100.

When the fourth step is performed, the first step, the second step, the third step, and the fourth step may be repeated multiple times in this order. This example allows the gas to be accurately discharged from the balloon catheter 20, and allows the gas to be accurately discharged from a portion of the liquid flow member 70, which serves as a liquid flow path to the balloon catheter 20. In addition, it can be avoided that a large amount of unused liquid 100 is accommodated in the reservoir accommodation chamber 80X of the reservoir 80. Namely, utilization efficiency of the liquid 100 can be improved, while the gas is more reliably discharged.

As described above, according to this embodiment, the gas 101 collected in the syringe 65 can be discharged to the liquid reservoir 80 easily and stably. The effect of reducing the burden of preparatory work becomes more significant by repeating the first step, the second step, the third step, and the fourth step.

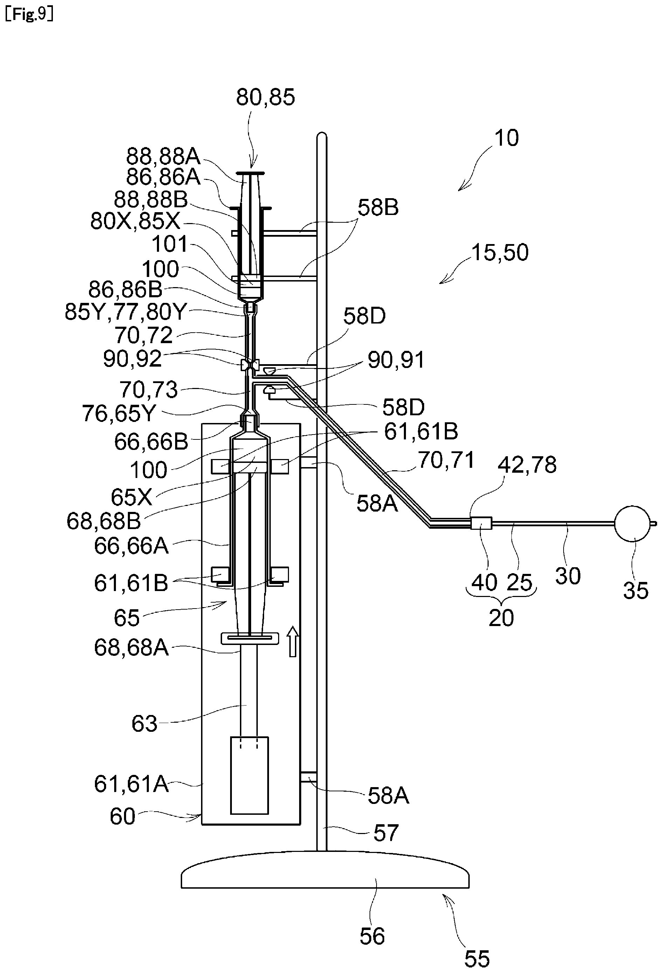

The balloon catheter 20 from which the gas has been discharged in this manner is inserted into a body of a patient. Prior to the insertion of the balloon catheter 20 into the body, the relative position of the outer shaft 31 and the inner shaft 32 is adjusted to a position at which the balloon 35 is stretched. After the balloon catheter 20 has been inserted into the body and the distal end of the balloon catheter 20 has been guided close to a target site (affected area), the relative position of the outer shaft 31 and the inner shaft 32 is adjusted to a position at which the balloon 35 can be inflated. Then, the inflation step shown in Fig. 9 is performed so that the balloon 35 is inflated.

In the conventional balloon catheter system, a surgeon or a technician supplies a suitable amount of the liquid to the balloon catheter to inflate the balloon 35 by a manual operation of the syringe. Thus, the conventional method cannot accurately adjust an amount of the liquid supplied to the balloon catheter. As a result, the size of the inflated balloon cannot be accurately controlled.

In this point, in the inflation step shown in Fig. 9, the liquid flow member 70 is placed in the first state. Then, by pushing the piston 68 into the cylinder 66 by means of the drive 63, a preset amount of the liquid 100 is supplied to the balloon catheter 20 to inflate the balloon 35. The drive 63 can adjust a relative movement amount of the piston 68 and the cylinder 66. This can adjust an amount of the liquid 100 supplied to the balloon catheter 20 so as to control the size of the inflated balloon 35. According to this inflation step, not a hand but the drive 63 operates the piston 68. Thus, the piston 68 can be relatively moved with respect to the cylinder 66 by a preset length and a desired length that is input each time. Namely, a suitable amount of the liquid 100 can be accurately supplied to the balloon catheter 20 to inflate the balloon 35 to an appropriate size.

Then, the preparation of the balloon catheter system 10 before procedure is completed, and a treatment step (heating step) is performed.

In the treatment step, the heating member 22 of the balloon catheter 20 is controlled by the power control unit 95A of the control device 95 to adjust a temperature of the liquid 100 in the balloon 35. Specifically, a high-frequency current is conducted from the power control unit 95A between the coil electrode 22A serving as the heating member 22 and the counter electrode 97 located outside the body of the patient. As a result, a high-frequency current is generated between the heating electrode 22A and the counter electrode 97. This allows the liquid 100 around the coil electrode 22A to be heated by Joule heat.

In the treatment step, the liquid flow member 70 is placed in the first sate, and the piston 68 is periodically moved to and from the cylinder 66 by means of the drive 63. The liquid 100 in the balloon 35 is heated, while the liquid 100 is repeatedly supplied to the balloon catheter 20 and is repeatedly discharged therefrom by the periodic relative movement of the piston 68 with respect to the cylinder 66. Namely, since the liquid 100 in the balloon 35 is heated while the liquid is vibrated and agitated, the temperature of the liquid 100 in the balloon 35 can be made uniform.

In the treatment step, a target site of the patient is ablated by bringing the balloon 35 containing the heated liquid 100 into contact with the target site.

As described in Patent Document 2 (JP 2010-233810 A), for example, the conventional balloon catheter system uses an agitator for agitating the liquid in the balloon, which is provided separately from a liquid supply device used for discharging the gas from the balloon catheter and for inflating the balloon. When the agitator is used, the discharge of the gas from the liquid flow path coupling the agitator and the balloon catheter must be performed separately from the discharge of the gas from the balloon catheter.

In this point, the aforementioned specific example of this embodiment can also discharge the gas from the liquid flow member 70 and the syringe pump 60 by discharging the gas from the balloon catheter 20. The liquid flow member 70 and the syringe pump 60 are used also for agitating the liquid 100 in the balloon 35. Such an embodiment can greatly facilitate the use of the balloon catheter system. In addition, it can also greatly simplify the structure of the balloon catheter system 10. This allows the operation using the balloon catheter system 100 to be performed more stably and more smoothly.

In addition, in the conventional balloon catheter system, as described in Patent Document 2 (JP 2010-233810 A), the agitator is typically a tube pump. The agitator using the tube pump can adjust cycle of the supply of the liquid and the discharge of the liquid. However, it is substantially difficult for the agitator using the tube pump to adjust a liquid amount. Thus, there are problems in that it takes time to make uniform the liquid temperature in the balloon, and that agitation conditions cannot be adjusted for each balloon state.

In this point, according to the aforementioned specific example of this embodiment, in the liquid control system 50, the drive 63 moves the piston 68 to and from the cylinder 66. The drive 63 can adjust an amplitude in the to-and-fro movement of the piston 68. Thus, in the treatment step in which the liquid 100 is heated, an amount of the liquid 100 to be supplied to the balloon catheter system 20 and an amount of the liquid 100 to be discharged therefrom can be varied by the drive 63 that adjusts the amplitude in the to-and-fro movement of the piston 68. Since the liquid amount can be adjusted, the liquid can be properly agitated in the balloon 35 to effectively avoid temperature variation of the liquid in the balloon 35.

In this manner, the target site is ablated. Upon completion of the ablation, the supply of energy to the heating member 22 is stopped. Then, the liquid flow member 70 is placed in the first state, and the liquid 100 is discharged from the balloon catheter 20 by suction of the syringe pump 60. This deflates the balloon 35. The deflated balloon 35 is stretched by relatively moving the outer shaft 31 and the inner shaft 32 to each other. Thereafter, the balloon catheter 20 with the stretched balloon 35 is pulled out from the body. In this manner, the use of the balloon catheter system 10 is completed. The balloon catheter assembly 15 pulled out from the body is discarded. The holding unit 61 and the drive 63 of the syringe pump 60 and the liquid flow controller 90 are again used together with the next supplied balloon catheter assembly 15.

In the aforementioned embodiment, the liquid control system 50 supplies the liquid to the balloon catheter 20 including the balloon 35 connected to the outer shaft 31 and the inner shaft 32, and discharges the liquid from the balloon catheter 20. The liquid control system 50 includes the syringe pump 60 including the syringe 65, the liquid flow member 70 coupling the syringe 65 and the balloon catheter 20, the reservoir 80 coupled to the syringe 65 by the liquid flow member 70, and the liquid flow controller 90 that controls flow of the liquid in the liquid flow member 70. The syringe pump 60 includes the syringe 65, the holding unit 61 holding the syringe 65, and the drive 63 that relatively moves the piston 68 with respect to the cylinder 66. The syringe 65 includes the cylinder 66, and the piston 68 that is relatively movable with respect to the cylinder 66. The liquid flow member 70 is connected to the cylinder 66 from above, and is connected to the reservoir 80 from below. The liquid flow controller 90 switches the first state in which the communication between the syringe 65 and the reservoir 80 is blocked and the communication between the syringe 65 and the balloon catheter 20 is allowed (achieved), and the second state in which the communication between the syringe 65 and the balloon catheter 20 is blocked and the communication between the syringe 65 and the reservoir 80 is allowed (achieved).

In the aforementioned embodiment, the using method of the balloon catheter system includes a step in which the liquid flow member 70 is placed in the first state, and the liquid in the syringe 65 is supplied to the balloon catheter 20 by pushing the piston 68 into the cylinder 66, and a step in which the liquid flow member 70 is placed in the first state, and the liquid and the gas in the balloon catheter 20 are discharged from the balloon catheter 20 by drawing out the piston 68 from the cylinder 66, and a step in which the liquid flow member 70 is placed in the second state, and at least a part of the liquid and the gas are sent to the reservoir 80 by pushing the piston 68 into the cylinder 66.

According to such an embodiment, the liquid can be supplied to the balloon catheter 20 and the liquid can be discharged from the balloon catheter 20 by means of the syringe pump 60. Namely, during the operation using the balloon catheter 20, the liquid in the balloon 35 can be agitated by using the syringe pump 60. Before the treatment, the syringe pump 60 used for the agitation can be used for discharging the gas from the balloon catheter 20. The gas discharged from the balloon catheter 20 is suctioned by the syringe pump 60. By switching the liquid flow member 70 from the first state to the second state, the gas discharged from the balloon catheter 20 can be sent to the reservoir 80. According to this embodiment, the liquid flow member 70 is connected to the syringe 65 from above, and is connected to the liquid reservoir 80 from below. Thus, the gas collected in the syringe 65 can be sent to the liquid reservoir 80 easily and stably. In addition, the syringe 65 can be held in a predetermined posture by the syringe pump 60, and the piston 68 can be accurately relatively moved with respect to the cylinder 66 at a desired amount. Further, along with the discharge of the gas from the balloon catheter 20, the gas can be discharged from the liquid flow member 70 serving as a liquid flow path between the balloon catheter 20 and the syringe 60 which can be used as an agitator of the balloon catheter 20. These features greatly reduce the burden of preparatory work before use of the balloon catheter system 10.

In the aforementioned specific example of the embodiment, the liquid flow controller 90 includes the first pinch valve 91 that opens and closes the first line part 71, and the second pinch valve 92 that opens and closes the second line part 72. The pinch valves 91, 92 can close the flow paths in the first line part 71 and the second line part 72, by bringing into contact with the fist line part 71 and the second line part 72 from the outside. Namely, the liquid flowing in the liquid flow member 70 is not exposed to the liquid flow controller 90. This avoids contamination of the liquid. In addition, the pinch valve has a simple structure with a solenoid, and can quickly open and close the flow path.

Note that, in place of this specific example, the liquid flow controller 90 may include a three-way valve connected to both the first line part 71 and the second line part 72. This simplifies the structure of the liquid flow controller 90.

In the aforementioned specific example of the embodiment, the reservoir 80 includes the reservoir syringe 85. The reservoir syringe 85 includes the cylinder 86 connected to the second line part 72, and the piston 88 that is relatively movable with respect to the cylinder 86. The cylinder 86 of the reservoir syringe 85 is positioned below the piston 68 of the reservoir syringe 85. The piston 88 of the reservoir syringe 85 is relatively moved upward and downward with respect to the cylinder 86 of the reservoir syringe 85. According to this specific example, since the reservoir accommodation chamber 80X of the reservoir 80, which accommodates the gas and the liquid, is delimited by the piston 88 and the cylinder 86, entering of foreign matter can be reduced. The volume of the reservoir accommodation chamber 80X increases or decreases depending on volumes of the gas and the liquid supplied thereto. Thus, the gas can be smoothly discharged from the balloon catheter 20, while avoiding contamination of the liquid.

In the aforementioned specific example of the embodiment, the holding unit 61 holds the syringe 65 such that the cylinder 66 is positioned above the piston 68. The drive 63 relatively moves the piston 68 upward and downward with respect to the cylinder 66. According to this specific example, in the accommodation chamber 65X of the syringe 65, the gas is positioned in an upper part such as the distal cylindrical part 66B. Thus, when the piston 68 starts movement to move the gas collected in the syringe 65 to the reservoir 80, the gas, prior to the liquid, is discharged from the syringe 65 toward the reservoir 80. This allows the gas collected in the syringe 65 to be stably guided to the reservoir 80. Namely, the gas can be stably discharged from the syringe 65. In the treatment step, the syringe pump 60 including the syringe 65 is used for agitating the liquid in the balloon 35. During the agitation of the liquid, the piston 68 is rapidly moved to and from the cylinder 66 to vibrate the liquid. By discharging the gas which can be compressed from the accommodation chamber 65X of the syringe 65 more reliably, the liquid can be stably vibrated.

One embodiment has been described with reference to the specific examples, but the aforementioned specific examples do to limit the embodiment. The aforementioned embodiment can be implemented in various other specific examples and can be variously omitted, replaced, changed, added, etc., within a scope that does not depart from the gist thereof.

The drive 63 may drive the piston 68 such that a movement speed at which the piston 68 is pushed into the cylinder 66 and a movement speed at which the piston 68 is drawn out from the cylinder 66 differ from each other. In the method of discharging the gas from the aforementioned balloon catheter 20, a relative movement speed (mm/sec) of the piston 68 with respect to the cylinder 66 in the first step (supply step) of supplying the liquid to the balloon catheter 20 may be slower than a relative movement speed of the piston 68 with respect to the cylinder 66 in the second step (discharge step) of discharging the liquid and the gas from the balloon catheter 20. According to this example, a supply speed (mL/sec) of the liquid to the balloon catheter 20 in the first step is less than a discharge speed (mL/sec) of the liquid from the balloon catheter 20 in the second step. This example allows the gas to be more reliably discharged from the balloon catheter 20 and the liquid flow member 70.

- 10: Balloon catheter system
- 15: Balloon catheter assembly
- 20: Balloon catheter
- 35: Balloon
- 50: Liquid control system
- 60: Syringe pump
- 61: Holding unit
- 63: Drive
- 65: Syringe
- 66: Cylinder
- 68: Piston
- 70: Liquid flow member
- 71: First line part
- 72: Second line part
- 73: Joint line part
- 76: Pump connection position
- 77: Reservoir connection position
- 78: Catheter connection position
- 80: Reservoir
- 85: Reservoir syringe
- 86: Cylinder
- 88: Piston
- 90: Liquid flow controller
- 91: First pinch valve
- 92: Second pinch valve

## Claims

1. A liquid control system that supplies a liquid to a balloon catheter and discharges the liquid from the balloon catheter, comprising:
a syringe pump including a syringe including a cylinder and a piston that is relatively movable with respect to the cylinder, a holding unit holding the syringe, and a drive that relatively moves the piston with respect to the cylinder;
a liquid flow member coupling the syringe and the balloon catheter;
a reservoir coupled to the syringe by the liquid flow member; and
a liquid flow controller that controls flow of the liquid in the liquid flow member;
wherein:
the liquid flow member is connected to the syringe from above, and is connected to the reservoir from below; and
the liquid flow controller switches a first state in which communication between the syringe and the reservoir is blocked and communication between the syringe and the balloon catheter is allowed, and a second state in which the communication between the syringe and the balloon catheter is blocked and the communication between the syringe and the reservoir is allowed.

2. The liquid control system according to claim 1, wherein:
the liquid flow member includes a first line part connected to the balloon catheter, and a second line part connected to the reservoir;
the liquid flow member including the second line part is connected to the cylinder from above; and
the second line part is connected to the reservoir from below.

3. The liquid control system according to claim 2, wherein
the liquid flow controller includes a first pinch valve that opens and closes the first line part, and a second pinch valve that opens and closes the second line part.

4. The liquid control system according to claim 2, wherein:
the reservoir includes a reservoir syringe including a cylinder connected to the second line part, and a piston that is relatively movable with respect to the cylinder; and
the cylinder of the reservoir syringe is positioned below the piston of the reservoir syringe, and the piston of the reservoir syringe is relatively moved upward and downward with respect to the cylinder of the reservoir syringe.

5. The liquid control system according to claim 1 wherein:
the holding unit holds the syringe such that the cylinder is positioned above the piston; and
the drive relatively moves the piston upward and downward with respect to the cylinder.

6. The liquid control system according to claim 1, wherein
a connection position between the liquid flow member and the syringe pump is positioned above a connection position between the liquid flow member and the balloon catheter.

7. The liquid control system according to claim 1, wherein
a connection position between the liquid flow member and the reservoir is positioned above a connection position between the liquid flow member and the syringe pump.

8. The liquid control system according to claim 1 wherein:
the liquid flow member includes a first line part connected to the balloon catheter, a second line part connected to the reservoir, and a joint line part connected to the syringe;
the first line part and the second line part branch off from the joint line part; and
a lowermost portion of the second line part is located at the same vertical position as an uppermost portion of the first line part, or at a position vertically above the uppermost portion of the first line part.

9. The liquid control system according to claim 1, wherein;
the liquid flow member includes a first line part connected to the balloon catheter, a second line part connected to the reservoir, and a joint line part connected to the syringe;
the first line part and the second line part branch off from the joint line part; and
a lowermost portion of the second line part is located at the same vertical position as an uppermost portion of the joint line part, or at a position vertically above the uppermost portion of the joint line part.

10. The liquid control system according to claim 1, wherein
the drive is capable of adjusting a relative movement amount of the piston and the cylinder.

11. The liquid control system according to claim 1, wherein
the drive drives the piston such that a movement speed at which the piston is pushed into the cylinder and a movement speed at which the piston is drawn out from the cylinder differ from each other.

12. A balloon catheter system comprising:
a liquid control system according to any one of claims 1 to 11; and
the balloon catheter.

13. A using method of a balloon catheter system according to claim 12, comprising:
a supply step in which the liquid flow member is placed in the first state, and the liquid in the syringe is supplied to the balloon catheter by pushing the piston into the cylinder;
a discharge step in which the liquid flow member is placed in the first state, and the liquid and gas in the balloon catheter are discharged from the balloon catheter by drawing out the piston from the cylinder; and
a transport step in which the liquid flow member is placed in the second state, and the gas is sent to the reservoir by pushing the piston into the cylinder.

14. The using method according to claim 13, wherein
the supply step, the discharge step, and the transport step are repeated in this order.

15. The using method according to claim 13, comprising a second discharge step following the transport step, in which the liquid flow member is placed in the second state, and the liquid is discharged from the reservoir by drawing out the piston from the cylinder, with the gas remaining in the reservoir.

16. The using method according to claim 13, comprising an inflation step following the transport step, in which the liquid flow member is placed in the first state, and a preset amount of the liquid is supplied to the balloon catheter to inflate the balloon by pushing the piston into the cylinder by means of the drive.

17. The using method according to claim 16, comprising a heating step following the inflation step, in which the liquid flow member is placed in the first state, and the liquid in the balloon is heated while supply of the liquid to the balloon catheter and discharge of the liquid therefrom are repeated by moving the piston to and from the cylinder by means of the drive.

18. The using method according to claim 16, wherein
the drive adjusts a relative movement amount of the piston and the cylinder.

19. The using method according to claim 13, wherein
a relative movement speed of the piston with respect to the cylinder in the supply step is slower than a relative movement speed of the piston with respect to the cylinder in the discharge step.

20. A balloon catheter assembly comprising:
a balloon catheter;
a liquid flow member connected to the balloon catheter;
a syringe including a cylinder coupled to the balloon catheter by the liquid flow member, and a piston that is relatively movable with respect to the cylinder; and
a reservoir coupled to the syringe by the liquid flow member;
wherein:
the liquid flow member enables communication between the syringe and the balloon catheter, and enables communication between the syringe and the reservoir; and
the liquid flow member is capable of being connected to the syringe from above, and is capable of being connected to the reservoir from below.
